# EUROPEAN PATENT SPECIFICATION

(11) **EP 1 543 785 B1**
(45) Date of publication and mention of the grant of the patent: **05.08.2009**
(21) Application number: 04257959.9
(22) Date of filing: 20.12.2004
(51) Int. Cl.: A61B 17/22

(54) **A basket-like medical treating tool for removing occlusive material**
Medizinisches Instrument mit Korb zur Entfernung von Okklusionen
Instrument médical en forme de panier pour l'extraction d'occlusions

(30) Priority: 18.12.2003 JP 2003420279; 18.02.2004 JP 2004040692
(43) Date of publication of application: 22.06.2005
(73) Proprietor: ASAHI INTECC CO., LTD., Nagoya-shi, Aichi-ken (JP)
(72) Inventor: Kato, Tomihisa, Aichi-ken (JP); Matsumoto, Munechika, Aichi-ken (JP); Kamei, Minekazu, Aichi-ken (JP)
(74) Representative: Price, Nigel John King

(56) References cited:
- DE-A1- 19 722 429
- FR-A- 2 808 991
- US-A- 4 046 150
- US-A1- 2002 165 557
- US-A1- 2003 055 445
- US-A1- 2003 109 889
- PATENT ABSTRACTS OF JAPAN vol. 1997, no. 05, 30 May 1997 (1997-05-30) & JP 09 019439 A (ASAHI OPTICAL CO LTD), 21 January 1997 (1997-01-21)

## Description

The invention relates to a medical treatment tool which is inserted into a human somatic cavity through an endoscope to retrievably capture foreign matters within the human somatic cavity as a therapeutic equipment to remove somatic foreign matters such as, for example, thrombi and the like from the somatic cavity.

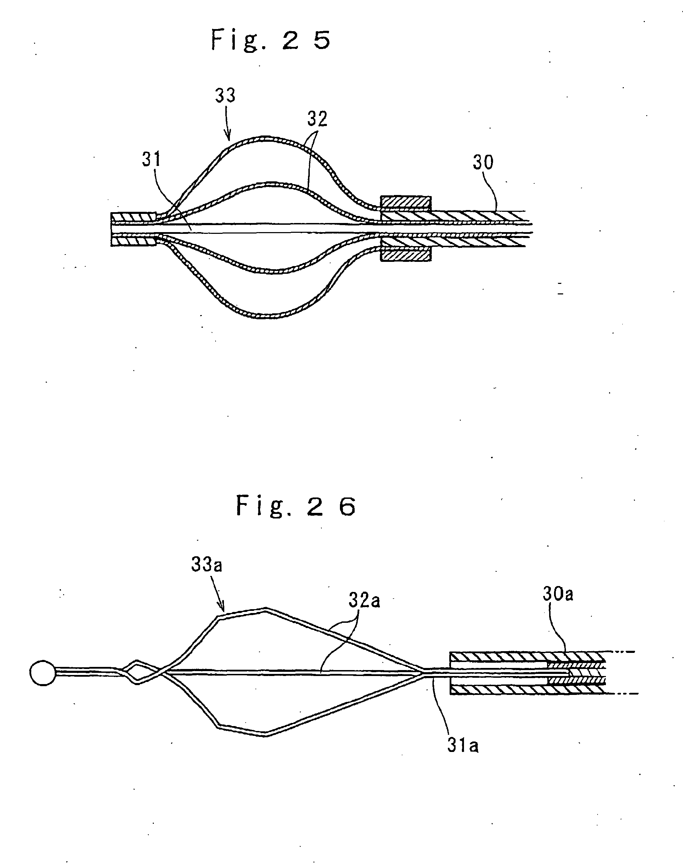

Medical treating tools used for retrievably capturing the foreign matters within the human somatic cavity are represented by Japanese Laid-open Patent Application Nos. 9-19439 and 2002-253558 (referred in turn to simply as "first reference and second reference" hereinafter). The first reference discloses a manipulation wire 31 slidably provided axially within a flexible plastic sheath 30 as shown in Fig. 25. At a distal end of the plastic sheath 30, a basket-shaped captor 33 is provided so that the basket-shaped captor 33 is diametrically expandable due to the action of several numbers of basket wires 32. By alternately expanding and shrinking the basket-shaped captor 33, it is possible to retrievably capture the foreign matters within the somatic cavity.

The second reference discloses a basket-shaped captor 33a defined by basket wires 32a which are curved by means of a die mould or heat treatment procedure as shown in Fig. 26. The basket-shaped captor 33a is secured to a distal end of a manipulation wire 31a which is slidably arranged within a sheath 30a. Together with an operation of the manipulation wire 31a, the basket-shaped captor 33a is alternately retracted into and protracted from the sheath 30a so as to be diametrically shrunken and expanded at the time when retrievably capturing the foreign matters to remove them from the somatic cavity.

In the basket-shaped captor 33 of the first reference, the basket-shaped captor 33 elastically shrinks into a predetermined original configuration when the manipulation wire 31 is released. The basket wires 32 are, however, structurally such as to render the basket-shaped captor 33 unable to tightly hold the foreign matters within the basket-shaped captor 33 so as to result in its retention capability being insufficient.

In this instance, the structure is such that the basket-shaped captor 33 is connectedly mounted on the plastic sheath 30. This makes the basket-shaped captor 33 likely to come off the sheath 30, while at the same time, thickening a connection area between the basket-shaped captor 33 and the sheath 30. The thickened area renders it difficult to make an endoscope structure thin, and thus increasing the burden on the subject patients when the medical tool is applied to the subject patients.

In the basket-like captor 33a of the second reference, the basket-shaped captor 33a is forcibly retracted into the sheath 30a when the manipulation wire 31a is pulled. In this situation, the retracting force easily enlarges a distal open end of the sheath 30a due to the physical resistance of the basket-shaped captor 33a against the sheath 30a. This renders the basket-shaped captor 33a unable to tightly hold the foreign matters within the basket-shaped captor 33 so as to result in its retention being unstable.

In the first and second references, the basket-shaped captor 33 (33a) is a bundle of basket wires 32 (32a) which generally forms a bow-shaped configuration with the manipulation wire 31 (31a) as a bowstring. This likely varies individual spaces between neighboring basket wires 32 (32a) to be larger or smaller to collapse the basket-shaped captor 33 (33a) far off its normal configuration due to the contact resistance induced when the basket-shaped captor 33 (33a) slides along the somatic cavity or the weight burden when the basket-shaped captor 33 (33a) captures the foreign matters.

As a result, the basket-shaped captor 33 (33a) makes it difficult for a manipulator to manoevreit to retrievably capture the foreign matters within the somatic cavity, and having a possibility to inadvertently drop the captured foreign matters within the somatic cavity, thus likely delaying the therapeutical treatment for the subject patients.

Additionally, the die mould and heat treatment procedure are adopted when deforming the wire coil elements into the basket-shaped captor 33 (33a), and thereafter the basket-shaped captor 33 (33a) is secured to the sheath 30 (30a) by means of a securement equipment. These complicated and time-consuming procudures are an impediment to forming the basket-shaped captor 33 (33a) precisely into an appropriate configuration.

Therefore, it is an object of the invention to overcome the above drawbacks, and provide a high quality medical treatment tool which is capable of eliminating the possibility to drop the captured foreign matters within the somatic cavity, thus making it possible to readily manoeuvre it to retrievably capture the foreign matters so as to facilitate the therapeutical treatment upon capturing the foreign matters.

US-A1- 2003/0055445 discloses a medical treatment tool on which the pre-characterizing portion of claim 1 is based.

US-A-4,046,150 discloses a medical treatment tool for removing occlusive objects which comprises a flexible tube or sheath which slidably receives a tightly-twisted multi-stranded central cable which can be extended beyond the sheath to form an expanding cage portion.

FR-A-2 808 991 discloses a medical treatment tool for removing occlusive objects which comprises a flexible tube or sheath which slidably receives an operation wire which extends beyond the sheath to the distal end of an expandable cage portion connected to the distal end of the sheath.

The present invention provides a medical treatment tool as defined by claim 1. Other preferred features are defined by the dependent claims and are explained below.

The medical treatment tool uses a wire-stranded helical hollow tube as a flexible linear wire by stranding a multitude of wire coil elements along a predetermined circle, and having an operational core elongation inserted into the wire-stranded helical hollow tube. After inserting the wire-stranded helical hollow tube into a somatic cavity, the operational core elongation is pulled to shift the diametrically expandable portion into a elastically expanded configuration so as to use it for a therapeutical treatment. By way of illustration, the diametrically expandable portion is formed by partly swaging an outer surface of the wire-stranded helical hollow tube as a diametrically reducing procedure.

The diametrically expandable portion functions to retrievably capture the foreign matters within the somatic cavity, and is formed from the wire-stranded helical hollow tube by exposing it from the outer protective layer. The wire-stranded hollow helical tube is continuous in one piece unitarily with the other part of the wire-stranded helical hollow tube which is coated with the outer protective layer. For this reason, the wire coil elements of the diametrically expandable portion staunchly respond to the pulling force appearing when the operational core elongation is pulled. This enables the manipulator to elastically deform the wire coil elements so as to shift the diametrically expandable portion into a basket-like configuration in which a bundle of ribs is inflated at its middle section with both ends of the ribs squeezed. With the release of the pulling force, the wire coil elements elastically shrinks to return the diametrically expandable portion to an original straight line configuration. Since the wire-stranded helical hollow tube except for the diametrically expandable portion is coated with the outer protective layer, the wire-stranded helical hollow tube except for the diametrically expandable portion is normally maintained straight with no risk of being expanded.

The wire coil elements of the wire-stranded helical hollow tube surround the operational core elongation in the spiral form with regular helical angles (lead angles) when the diametrically expandable portion is elastically expanded with the residual stress maintained in the wire coil elements helically stranded. This significantly reduces the strain appeared on the wire coil elements when subjected to an exterior force. The reduced strain level makes it possible to keep a normal space between the neighboring wire coil elements without collapsing the wire coil elements when the wire coil elements are elastically expanded to shift the diametrically expandable portion into a basket-like configuration, an outer contour of which is defined by the wire coil elements spirally deformed. Thus the diametrically expandable portion maintains the normal basket-like configuration without collapsing even when subjected to the contact and viscous resistance met within the somatic cavity. This imparts the basket-like configuration with an appropriate rigidity enough to tightly hold the captured foreign matters in the diametrically expandable portion.

With the basket-like configuration provided by a bundle of the wire coil elements formed into the bow-shaped configuration with the operational core elongation as a bowstring, it is possible to adjust a dimensional length of the bowstring due to a relative sliding displacement against the wire-stranded helical hollow tube. The adjustment of the bowstring enables the manipulator to shift the basket-like configuration into a flat-shaped formation diametrically greater with a smaller span, a spindle-shaped formation diametrically smaller with a greater span and a medium-sized formation positioned between the former two formations.

In addition, the structure is such that the diametrically expandable portion can be placed at any position along the main linear wire portion. This provides the diametrically expandable portion with a multitude of formation patterns appropriately selectable depending on the diseased area within the somatic cavity. Due to the formations characteristic of the diametrically expandable portion, it is possible to readily capture the foreign matters within the somatic cavity, and further prevent the captured foreign matters from inadvertently dropped.

By shifting the basket-like configuration into the flat-shaped formation after capturing the foreign matters with the basket-like configuration in the spindle-shaped formation or the medium-sized formation, the resultant formation increases an inclination angle of the wire coil elements against the operational core elongation, and increasing the contact area against the foreign matters to tightly hold them within the basket-like configuration so as to therapeutically remove the foreign matters more appropriately.

With at least the diametrically expandable portion of the wire-stranded helical hollow tube reduced by means of swaging or die mould procedure, the wire coil elements of the diametrically expandable portion are forced to tightly engage each other to have a fan-shaped cross section. This imparts the wire coil elements with proper rigidity to hold the somatic foreign matters within the basket-like configuration more tightly than the related art counterpart can afford. With the wire coil elements reduced by means of swaging or die mould procedure to make them more rigid, it is possible to further thin the wire coil elements without losing the appropriate holding capability against the foreign matters, while at the same time, thinning the endoscope to mitigate the burden which the subject patients suffers from.

With the medical treatment tool having the operational core elongation and the wire-stranded helical hollow tube coated with the outer protective layer as main constituents, it is possible to simplify the whole structure to stabilize the mechanical structure easy to produce more than the related art counterpart can afford with the constituents of the sheath, the operational core elongation and the basket wires.

On the other hand, the related art counterpart has to add a bending tendency to the basket wires and requires the heat treatment to maintain the bending tendency for the basket wires. This inevitably complicates the procedures to bundle the basket wires and array them around the operational core elongation. The complication renders it difficult to produce the basket-like configuration precisely and involving a risk of misarranging the basket wires with less stable formation.

On the contrary, according to the subject invention, only by separatively removing the outer protective layer from the wire-stranded helical hollow tube, it is possible to precisely produce the diametrically expandable portion. It enables manufacturers to select a desired position for the basket-like configuration by simply changing the place in which the outer protective layer is removed.

.

The diametrically expandable portion can be axially severed at its wire coil elements to form a spoon-like configuration with its open end surface as a wire-lost region. In this instance, the diametrically expandable portion introduces a new efficient usage to function as a ladle to capture the somatic foreign matters when shifted into the spoon-like configuration within the somatic cavity.

According to other aspect of the present invention, a dimensional length of wire coil elements of the diametrically expandable portion is preferably approximately equal to an axial span appeared when the wire coil elements are angularly twisted by 360 degrees.

The medical treatment tool thus described above enables the manipulator to retrievably capture the foreign matters quickly and unerringly within the somatic cavity, and whereby significantly enhancing the curability with high practicability.

Preferred forms of the present invention are illustrated in the accompanying drawings in which:
Fig.1 is a plan view of a medical treatment tool according to a first embodiment of the invention;
Figs. 2, 3 and 4 are longitudinal cross sectional views of a main part of the medical treatment tool;
Fig. 5 is a latitudinal cross sectional view of a wire-stranded helical hollow tube;
Fig. 6 is a plan view of a diametrically expandable portion;
Fig. 7 is a latitudinal cross sectional view of an operational core elongation;
Figs. 8, 9 and 10 are schematic views depicting how the medical treatment tool works;
Fig. 11 is a plan view of a medical treatment tool according to a second embodiment of the invention;
Fig. 12 is a plan view of a main part of the medical treatment tool;
Fig. 13 is a latitudinal cross sectional view of a main part of an operational core elongation;
Fig. 14 is a schematic view depicting how the medical treatment tool works;
Fig. 15 is a schematic view depicting how a medical treatment tool works according to a third embodiment of the invention;
Fig. 16 is a longitudinal cross sectional view of a main part of a medical treatment tool according to a fourth embodiment of the invention;
Fig. 17 is an enlarged plan view of a diametrically expandable portion according to a fifth embodiment of the invention;
Fig. 18 is a latitudinal cross sectional view of a wire-stranded helical hollow tube;
Fig. 19 is a latitudinal cross sectional view of a wire-stranded helical hollow tube according to a sixth embodiment of the invention;
Fig. 20 is a latitudinal cross sectional view of a wire-stranded helical hollow tube according to a seventh embodiment of the invention;
Fig. 21 is a longitudinal cross sectional view of a diametrically expandable portion according to an eighth embodiment of the invention;
Fig. 22 is a plan view of a main part of a medical treatment tool according to a ninth embodiment of the invention;
Fig. 23 is a plan view of a medical treatment tool depicting how it works;
Fig. 24 is a plan view of a main part of a medical treatment tool according to a tenth embodiment of the invention; and
Figs. 25 and 26 are longitudinal cross sectional views of a main part of a related art treatment tool.

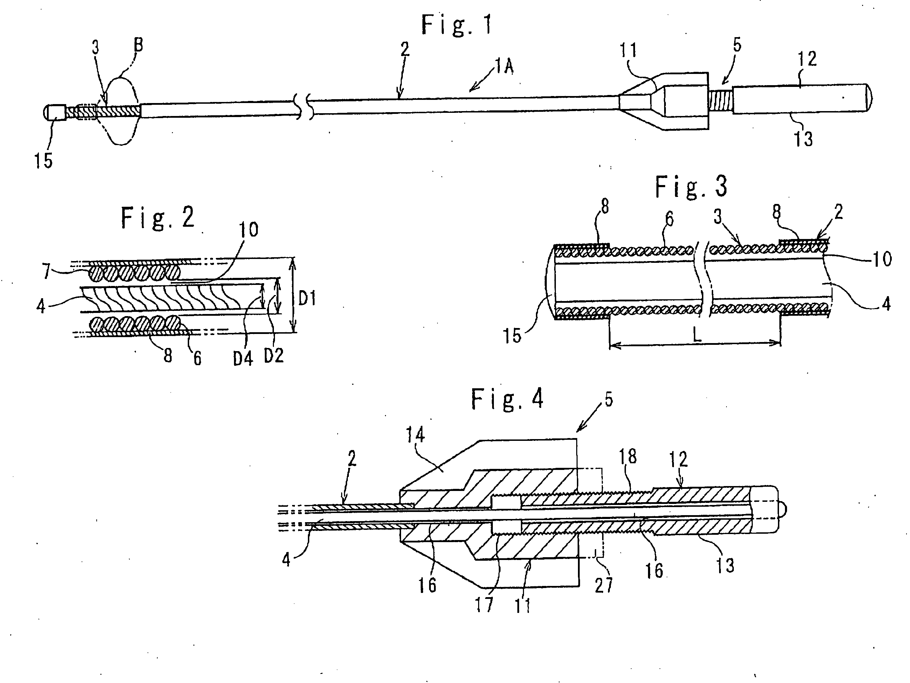

In the following description of the depicted embodiments, the same reference numerals are used for features of the same type. Referring to Figs. 1 through 10, a medical treatment tool 1A (abbreviated as "treatmeant tool 1A" hereinafter) according to a first embodiment of the invention is described below.

In the treatment tool 1A as shown in Fig. 1, a diametrically expandable portion 3 is provided in proximity of a distal end of a main linear wire portion 2 which acts as a flexible linear wire. The diametrically expandable portion 3 forms into a basket-like configuration (B) to retrievably capture foreign matters within a human somatic cavity as described in detail hereinafter. At a rear end of the main linear wire portion 2, a hand access portion 5 is provided to actuate the diametrically expandable portion 3 to shift into the basket-like configuration (B).

In this instance, the main linear wire portion 2 is defined into a wire-stranded helical hollow tube 6 by stranding a multitude of wires around a core member (withdrawn later) or stranding a multitude of wires along a predetermined circle line with a spatial bore 10 left in the axial direction as shown in Figs. 2 and 3. An outer surface of the main linear wire portion 2 is coated with an outer protective layer 8 which is made of synthetic resin material.

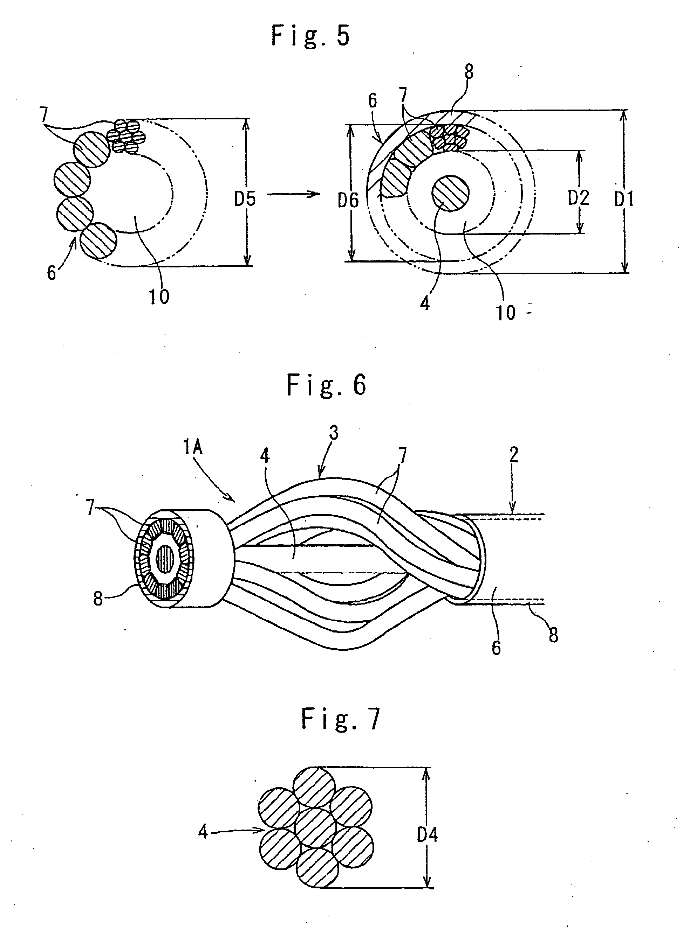

Upon providing the wire-stranded helical hollow tube 6 (see Fig. 5), seven thin wires (0.1 mm in diameter) are twisted to form a wire coil element 7 (0.3 mm in diameter) so that the ten wire coil elements 7 are stranded to produce a hollow helical coil body (1.3 mm in diameter D5). Then, the hollow helical coil body is dimensionally reduced at its outer surface to be 1.1 mm in diameter (D6) by means of swaging or die mould procedure. This renders the wire coil elements 7 to have a fan-like latitudinal cross section with the neighboring wire lines tightly engaging each other. Coating the outer protective layer 8 makes the hollow helical coil body to be 1. 5 mm in outer diameter (D1) and 0.7 mm in inner diameter (D2).

For coating the outer protective layer 8, the thermoplastic material (e.g., polyamide- or fluoro-based resin) or the thermosetting material (e.g., phenol- or epoxy-based resin) can be used. An extrusion procedure or a heat-shrinkable tube may be used when providing the outer protective layer 8 on the hollow helical coil body. Preferably, the outer protective layer 8 has a R-scale number more than 80 in terms of Rockwell's hardness especially when the thermoplastic material is used to the outer protective layer 8.

The main linear wire portion 2 places an operational core elongation 4 slidably in the axial direction within the spatial bore 10. A distal end of the operational core elongation 4 is connected to a distal end of the main linear wire portion 2 (see Fig. 3), and a rear end of the operational core elongation 4 is connected to a male thread segment 12 provided at the hand access portion 5 (see Fig. 4). The diametrically expandable portion 3 reveals by separatively removing the outer protective layer 8 so as to straightly expose the wire-stranded helical hollow tube 6 (extending by L mm in length). To the distal end of the the main linear wire portion 2, a head plug 15 is secured which is connected to a distal end of the operational core elongation 4. The hand access portion 5 has a bar-shaped grip 13 which has a central bore 16 to slidably place the operational core elongation 4 (see Fig. 4) within it.

The hand access portion 5 further has a screw mechanism composed of a cylindrical female thread portion 11 and a male thread portion 12. The female thread portion 11 forms a female thread hole 17, and the male thread portion 12 provides a male thread bar 18 at an forward tip of the grip 13 to be screwed into the female thread hole 17. From both sides of the female thread portion 11, a fin plate 14 extends outward along a common flat plane.

Upon rotating the male thread portion 12 (grip 13) by finger tips while holding the fin plate 14 by other finger tips, the rotational manipulation gives the operational core elongation 4 a pulling force to slide the operational core elongation 4 within the wire-stranded helical hollow tube 6, thus realizing the relative displacement of the operational core elongation 4 against the wire-stranded helical hollow tube 6.

In this instance, the operational core elongation 4 is produced by seven line wires (0.16 mm in diameter) as shown in Fig. 7. Among the seven line wires, the six line wires are stranded around the rest of the line wires (1 × 7) to form a twisted wire structure (approx. 0.5 mm in diameter D4).

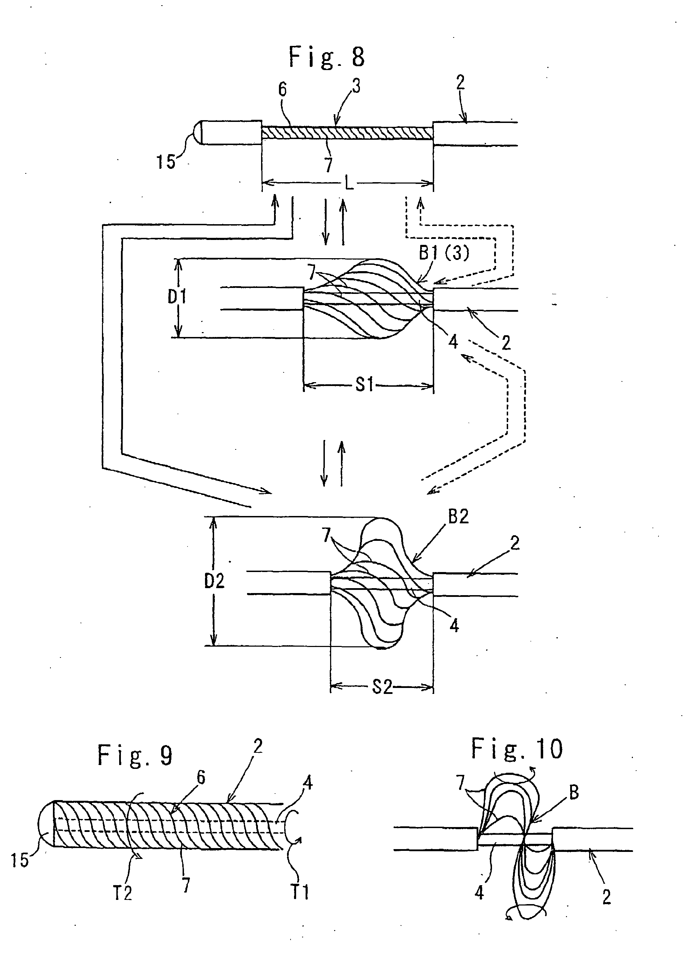

The structure of the screw mechanism is such that the rotation of the male thread portion 12 (in the left direction for a right screw, and in the right direction for a left screw) retracts the male thread portion 12 to pull the operational core elongation 4. For this reason, the stranding direction of the operational core elongation 4 is such as to be tightly wound (s-strand, z-strand) when subjected to the rotation in the pulling direction (see Fig. 9). The stranding direction of the wire-stranded helical hollow tube 6 is the same as that of the operational core elongation 4.

Upon inserting the treatment tool 1A into the somatic cavity to retrievably capture the foreign matters within the somatic cavity, the hand access portion 5 is rotationally manipulated to slidably pull the operational core elongation 4 rearward through the main linear wire 2. The pulling force gives the head plug 15 an attraction force toward the hand access portion 5 to make the diametrically expandable portion 3 function as a shock absorbing cushion for the pulling force.

Then, the attraction force exerts against the diametrically expandable portion 3 to elastically deform the wire coil elements 7 outward into a mountain-like configuration as directed by the dotted arrow line in Fig. 8. During this transition, the wire coil elements 7 curves outward with regular intervals appeared between the neighboring wire coil elements 7 (as contoured rib lines) so as to inflate the diametrically expandable portion 3 into a basket-like configuration (B1) at a larger diameter (D1) with a greater span (S1).

In this situation, the hand access portion 5 is manipulated to release the pulling force so that the wire coil elements 7 liberates its elastic restitution to permit the relative movement between the operational core elongation 4 and the main linear wire portion 2. Namely, the elastic restitution of the wire coil elements 7 moves the operational core elongation 4 forward or retracts the main linear wire portion 2 so as to return the basket-like configuration (B1) to the original shrunken configuration (or pseudo-original position) with the wire coil elements 7 substantially placed at the original straight configuration.

Upon treating the diseased area within the somatic cavity, the diametrically expandable portion 3 is manipulated to retrievably capture the foreign matters at the basket-like configuration (B1). In order to prevent the captured foreign matters from inadvertently dropped, the hand access portion 5 is manipulated to adjust the pulling degree against the operational core elongation 4.

This further inflates the diametrically expandable portion 3 from the basket-like configuration (B1) at the larger diameter (D1) with the greater span (S1) to another basket-like configuration (B2) at a still larger diameter (D2) with a yet greater span (S2) as directed by the solid arrow line in Fig. 8. With the use of the basket-like configuration (B2), it is possible to make the wire coil elements 7 tightly hold the foreign matters so as to remove them unerringly from the somatic cavity. It is to be noted that it is sufficient to reduce only the diametrically expandable portion 3 instead of reducing an entire length of the wire-stranded helical hollow tube 6.

With the structure thus far described, the wire coil elements 7 of the diametrically expandable portion 3 has the fan-like cross sectional configuration so that the wire coil elements 7 (cornered edge portion) works as a pulverlizer against the foreign matters captured by the diametrically expandable portion 3.

With the diametrically expandable portion 3 progressively shrunken (see Fig. 8), the wire coil elements 7 reversely curves perpendicular to the operational core elongation 4 so as to more tightly hold the captured foreign matters as shown at arrows in Fig. 10.

The operational core elongation 4 and the wire-stranded helical hollow tube 6 are stranded such that the operational core elongation 4 is not subjected to the force in the unwinding direction when the operational core elongation 4 is manipulatively rotated to be pulled. This provides the operational core elongation 4 with a good transmissibility against the pulling operation. The wire-stranded helical hollow tube 6 is rotationally influenced as an reaction in the direction opposite to the arrow T2 as shown in Fig. 9 when the operational core elongation 4 is rotated to be pulled in the direction as shown by the T1. The wire-stranded helical hollow tube 6 exerts its rotational influence to works against the diametrically expandable portion 3 in the unwinding direction so as to help inflate the diametrically expandable portion 3.

The wire-stranded helical hollow tube 6 of the diametrically expandable portion 3 has the wire coil elements 7 spirally twisted and stranded. This provides the wire coil elements 7 with a good pliability compared to the counterpart structure in which the straight line wires are only unitedly bundled. This also increases the contact surface area of the wire coil elements 7 against the foreign matters, thus making it possible to retrievably capture the thrombi and viscous gore efficiently in the blood vessel without doing harm on the vascular wall. Thus, the treatment tool 1A enables the manipulator to readily capture the somatic foreign matters unerringly so as to significantly enhance the curability against the diseased lesion.

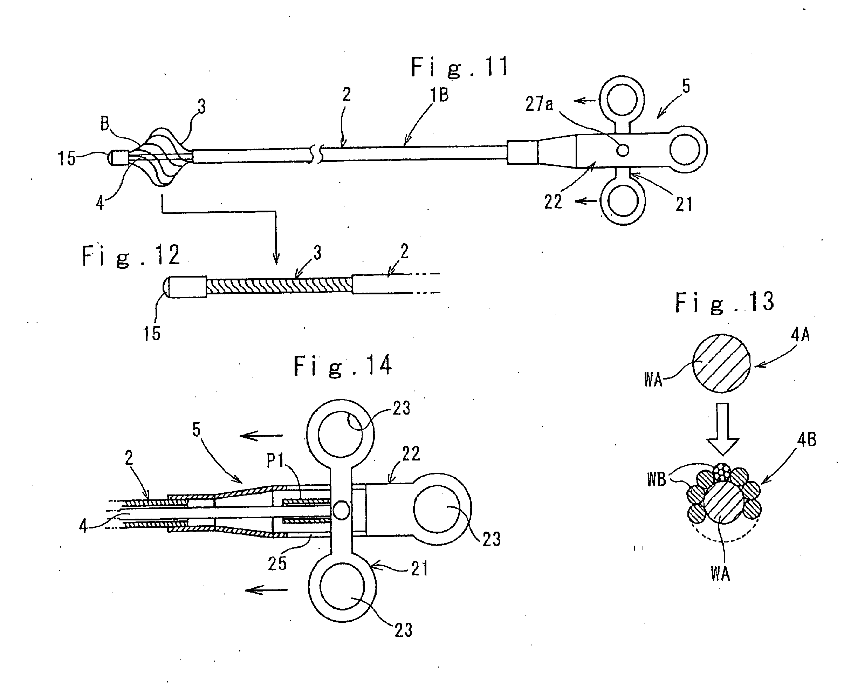

Figs. 11 through 14 show a second embodiment of the invention in which a medical treatment tool 1B is provided (abbreviated as "treatment tool 1B" hereinafter). The treatment tool 1B has the main linear wire portion 2, the diametrically expandable portion 3, the operational core elongation 4, the hand access portion 5 and the wire-stranded helical hollow tube 6 in the same manner as the first embodiment of the invention. The treatment tool 1B differs structurally from the treatment tool 1A in that the diametrically expandable portion 3 is plastically deformed in unrestricted free state into the basket-like configuration inflated to such a degree as shown by the notation (B1) in Fig. 8.

Upon deforming the diametrically expandable portion 3, the wire coil elements 7 of the wire-stranded helical hollow tube 6 is placed into a spiral groove provided with a barrel-shaped mould (not shown) after separatively removing the outer protective layer 8 from the main linear wire 2. Then, the wire coil elements 7 is wrought out by an upper and lower mould (not shown) depressed on the barrel-shaped mould. Thereafter, the wire coil elements 7 are thermally treated on the barrel-shaped mould, and removed from the barrel-shaped mould to maintain the diametrically expandable portion 3 in the basket-like configuration (see Fig. 11).

The push operation against the operational core elongation 4 moves it forward to forcibly shrink the diametrically expandable portion 3 into a linear straight configuration or slightly bulged configuration which is dimensionally enough to be inserted into the somatic cavity (see Fig. 12). The pull operation against the operational core elongation 4 makes the diametrically expandable portion 3 deform into the basket-like configuration inflated to such a degree as shown by the notation (B2) in Fig. 8.

The hand access portion 5 has a tubular male segment 22 connected to a rear end of the main linear wire portion 2 as shown in Fig. 14. The male segment 22 has a circumferential wall at a tubular portion in which an axial slit 25 is provided through which a female segment 21 is slidably disposed perpendicular to the male segment 22. A central portion of the female segment 21 is connected to the rear end of the operational core elongation 4. The female segment 21 has two finger holes 23 at both ends while the male segment 22 has one finger hole 23 at the rear extremity end. The female segment 21 and the male segment 22 constitute a slide mechanism arranged to move near and apart each other.

With the finger tips put into the finger holes 23, the push operation against the female segment 21 relatively moves the operational core elongation 4 through the main linear wire portion 2. This push operation makes the diametrically expandable portion 3 shrink into the linear straight configuration in the same manner as directed by the dotted arrow line in Fig. 8 in which the diametrically expandable portion 3 deforms from the basket-like configuration (B1). The treatment tool 1B is inserted into the somatic cavity to manipulatively deform the diametrically expandable portion 3 into the basket-like configuration (B1, B2) in the same manner as described in the first embodiment of the invention.

In this instance, the operational core elongation 4 is provided by a thick line core WA or thin line wires WB stranded around the thick line core WA to have high anti-buckling property as represented by numerals 4A and 4B in Fig. 13. This is to cope with the contractile force applied to the operational core elongation 4 in the lengthwise direction when manipulatively pushed. By the same token, the female segment 21 has a buckle-preventive pipe P1 around the operational core elongation 4 as shown in Fig. 14. The tubular portion of the male segment 22 has a set lock screw 27a to hold the diametrically expandable portion 3 in an appropriately inflated configuration.

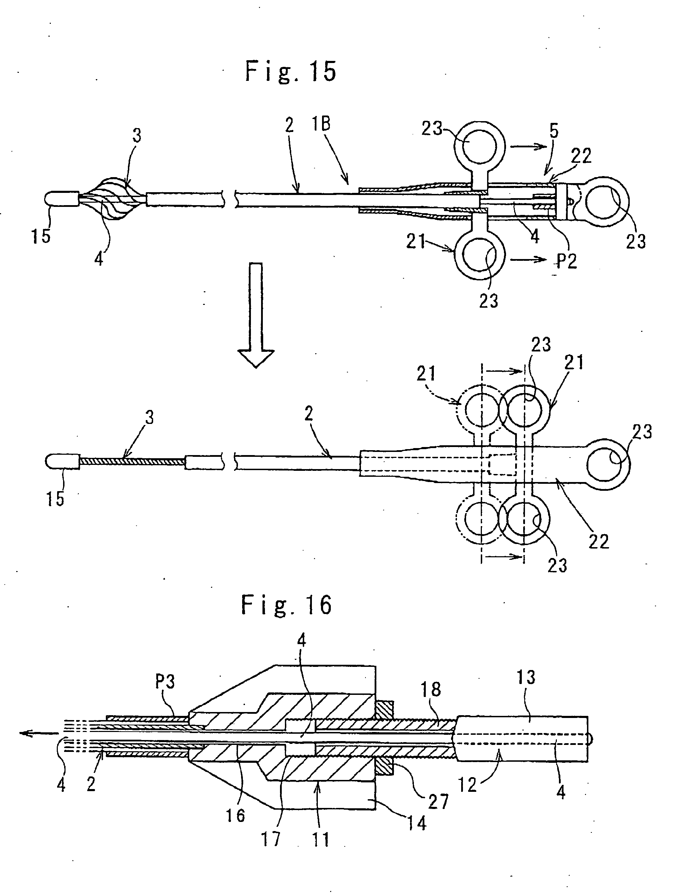

Fig. 15 shows a third embodiment of the invention in which the hand access portion 5 has the female segment 21 and the male segment 22 arranged in the same manner as described in the second embodiment of the invention. The rear end of the operational core elongation 4 pierced through the central portion of the female segment 21 and connected to the male segment 22. The female segment 21 is secured to the main linear wire 2 to permit the relative movement against the operational core elongation 4.

With the push operation of the female segment 21 against the male segment 22, the main linear wire 2 retracts relative to the operational core elongation 4 to deform the diametrically expandable portion 3 into an appropriately inflated configuration (expandable process). In order to prevent the operational core elongation 4 from inadvertently buckled when pulling the female segment 21 toward the male segment 22, an anti-buckling pipe P2 is secured to the male segment 22 in the manner to surround the operational core elongation 4. During the expanding process of the diametrically expandable portion 3, the same manipulation is taken as the related art counterpart. This can be useful in that the manipulator can utilize his or her skilled technique already acquired.

Fig. 16 shows a fourth embodiment of the invention in which the hand access portion 5 has the same type structure as the treatment tool 1A has in the first embodiment of the invention. The rear end of the main linear wire portion 2 is connected to the front end of the female thread portion 11 while the rear end of the operational core elongation 4 connected to the rear end of the male thread portion 12. The rotational manipulation of the male thread portion 12 moves (pushes) the operational core elongation 4 forward so as to deform the diametrically expandable portion 3 in the shrinkable direction. As occasion requires, the male thread portion 12 can be reversely rotated to pull the operational core elongation 4 so as to deform the diametrically expandable portion 3 in the inflatable direction. To the front end of the female thread portion 11, an anti-buckling pipe P3 is secured to protect the main linear wire 2 against the contractile stress applied to the main linear wire 2 when rotated in the pushing direction.

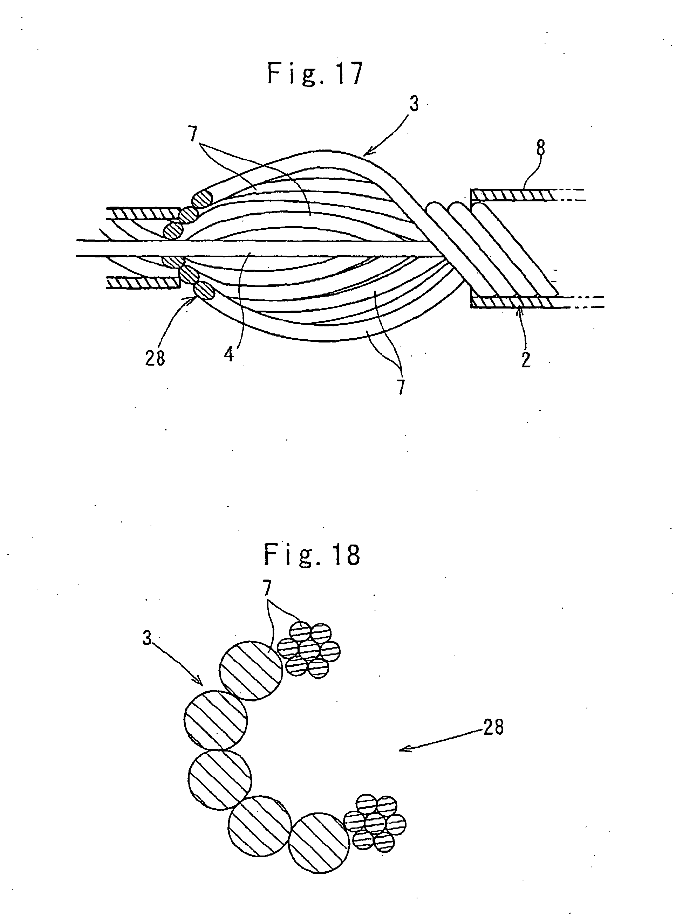

Figs. 17 and 18 show a fifth embodiment of the invention in which the diametrically expandable portion 3 deforms into a spoon-like configuration. Among the wire coil elements 7 of the wire-stranded helical hollow tube 6 at the diametrically expandable portion 3, several numbers (e.g., three) of the ten wire coil elements 7 are removed from the wire-stranded helical hollow tube 6 to provide a wire-lost region 28 which functions as an open entrance for capturing the foreign matters when inflated into the spoon-like configuration.

Within the somatic cavity, the manipulation rotates the diametrically expandable portion 3 which deforms a shallow-bottom spoon configuration to a deep-bottom configuration. This makes the diametrically expandable portion 3 serve as a ladle to efficiently scoop the somatic foreign matters. The spoon-like configuration makes it easy to pass the diametrically expandable portion 3 through the bifurcated portion of the blood vessel and the digestive organs so as to enhance the performance with high quality. It is to be noted that the wire coil elements 7 is preferably formed by the highly rigid wire structure as shown in Fig. 13 to cope with greater stresses applied to the wire coil elements 7 when the diametrically expandable portion 3 is manipulated as the ladle.

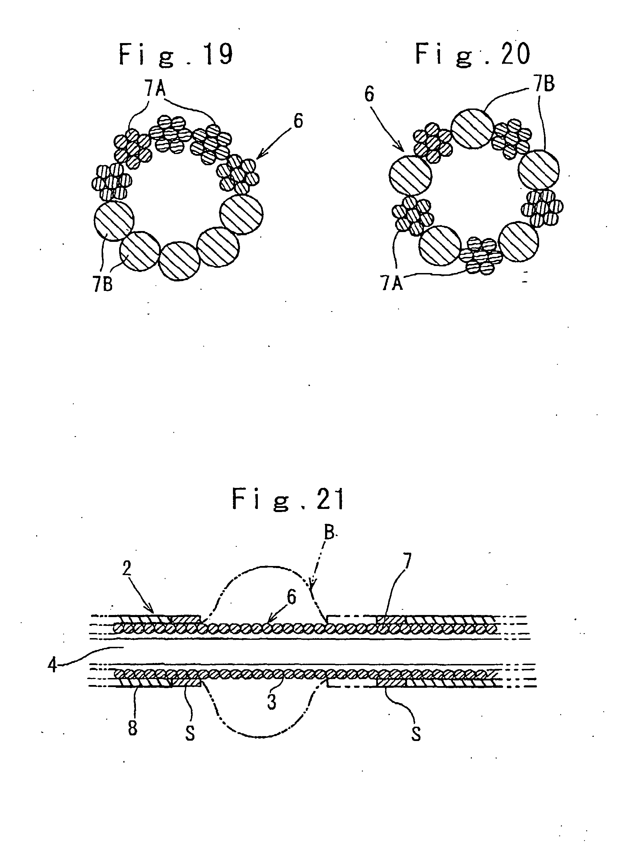

Fig. 19 shows a sixth embodiment of the invention in which the wire coil elements 7 of the diametrically expandable portion 3 has an exotic structure. Six single line wires 7B (rigid wires) and four stranded line wires 7A (1 × 7 type pliable wires) are prepared. The six consecutive single line wires 7B and the four consecutive stranded line wires 7A are twisted to form the wire-stranded helical hollow tube 6.

Fig. 20 shows a seventh embodiment of the invention in which the single line wires 7B and the stranded line wires 7A are twisted alternately.

With the structure of the sixth and seventh embodiment of the invention, it is possible to elastically inflate the wire coil elements 7 against the viscous resistance under the presence of the rigid wires 7B even if the wire coil elements 7 are stuck in the viscous secretion upon manipulating the diametrically expandable portion 3 within the somatic cavity.

It is also possible to inflate the wire coil elements 7 at equal intervals between the neighboring coil elements under the presence of the rigid wires 7B even if the wire-stranded helical hollow tube 6 encounters the concave-convexed wall of the human organs. The pliable wires 7A makes it easy to deform the wire-stranded helical hollow tube 6 outward to let the somatic foreign matters readily enter into the diametrically expandable portion 3. Due to the rigidity differentiated between a group of the pliable wires 7A and a group of the rigid wires 7B (see Fig. 19), the wire-stranded helical hollow tube 6 is likely to bend preferentially in one direction so as to favorably lead the wire-stranded helical hollow tube 6 into the bifurcated portion within the blood vessel and the organs.

In the sixth and seventh embodiment of the invention, the length dimension (L) of wire coil elements 7 of the diametrically expandable portion 3 is approximately equal to an axial span appeared when the wire coil elements 7 are angularly twisted by 360 degrees (i.e., one single lead pitch). In this instance, the wire coil elements 7 deforms to turn by 360 degrees around the axial extension of the diametrically expandable portion 3 when inflated into the basket-like configuration (B). This makes it possible to efficiently capture the somatic foreign matters especially when inflated into the basket-like configuration (B2) at the larger diameter (D2) with the greater span (S2) as described in Fig. 8. The further pull operation against the operational core elongation 4 makes the wire coil elements 7 engage with each other so as to build up the holding capacity for the captured foreign matters.

Fig. 21 shows an eighth embodiment of the invention in which a unity ring band S is provided as a fixation member in the treatment tool 1A (1B). The unity ring band S is fittingly secured to both ends of the diametrically expandable portion 3 in order to physically unite these end portions. Instead of using the unity ring band S, both the ends of the diametrically expandable portion 3 may be welded by means of a suitable welding means.

With the fixation member secured to these end portions, the fixation member bears the stress applied to the ends of the diametrically expandable portion 3 when the diametrically expandable portion 3 deforms into the basket-like configuration (B), thus making it possible to prevent the wire coil elements 7 from coming loose divergently at the ends of the diametrically expandable portion 3. This stabilizes the deformation of the diametrically expandable portion 3 without collapsing the basket-like configuration (B) even if repeatedly deform the diametrically expandable portion 3. The stable deformation enables the manipulator to more stably capture the foreign matters within the somatic cavity.

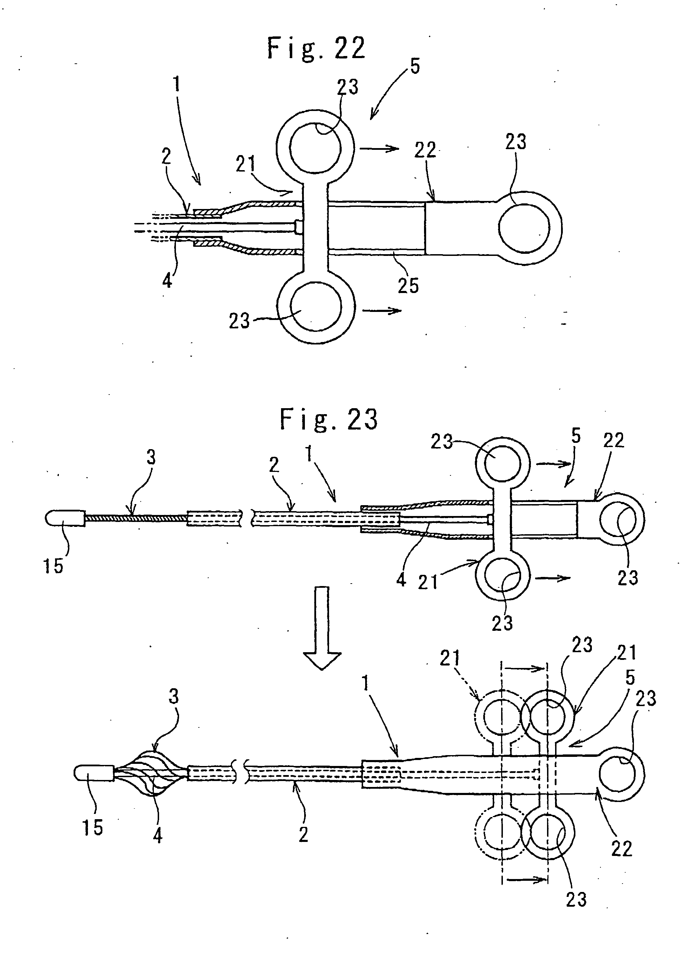

Figs. 22 and 23 show a ninth embodiment of the invention which has the main linear wire portion 2, the operational core elongation 4 and the diametrically expandable portion 3 as the main structure as described in the first embodiment of the invention.

The hand access portion 5 has the slide mechanism by the combination of the female segment 21 and the male segment 22 as shown in Fig. 22. The tubular male segment 22 is connected at its front end to the rear end of the main linear wire portion 2. The rear end portion of the male segment 22 has the finger hole 23 which is also provided at both distal wings of the female segment 21. The female segment 21 is crosswisely inserted into the slit 25 of the male segment 22 to slidably move along the the male segment 22. The rear end of the operational core elongation 4 is connected to the central portion of the female segment 21 to move in association with the female segment 21.

With the finger tips put into the finger holes 23 as shown in Fig. 23, the female segment 21 is pulled along the male segment 22 to retract the operational core elongation 4 sliding through the main linear wire portion 2. In combination with the pull operation, the head plug 15 pushes the diametrically expandable portion 3 in the contractile direction so as inflate the diametrically expandable portion 3 into the basket-like configuration (B1, B2).

In this instance, as far as the deformation of the diametrically expandable portion 3 is concerned, the pull operation against the female segment 21 oppositely functions to the pull operation which the treatment tool 1B works in Fig. 15. It is to be noted that the lock screw may be placed as the lock member to adjustably hold the female segment 21 at the desired position against the male segment 22.

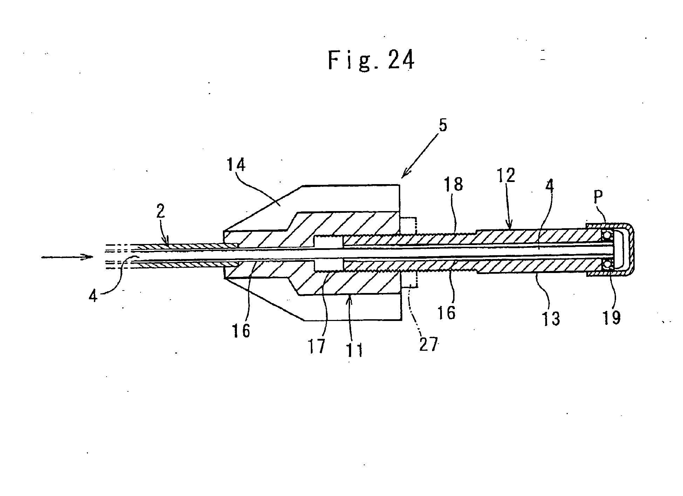

Fig. 24 shows a tenth embodiment of the invention in which the screw mechanism is provided at the hand access portion 5 by the female thread portion 11 and the male thread portion 12 in the same manner as described at the first embodiment of the invention. The operational core elongation 4 is slidably placed within the central bore 16 which is pierced through the female thread portion 11 and the male thread portion 12. To the rear end of the grip 13, a finger cap P is rotationally secured against the male thread portion 12 by means of a roller bearing 19. The finger cap P moves around the male thread portion 12 as a rotational force relieve member to release harmful outer force inadvertently applied to the operational core elongation 4 when the male thread portion 12 is rotationally operated.

The finger cap P is useful particularly when the mechanical design restricts the operational core elongation 4 to rotationally move in such a direction as to tightly wind the wire coil elements 7 upon manipulatively rotating the grip 13 in the direction that the operational core elongation 4 is pulled.

### MODIFICATION FORMS

It is to be noted that the hand access portion 5 of the treatment tool 1B may be secured to the treatment tool 1A, and conversely, the hand access portion 5 of the treatment tool 1A may be secured to the treatment tool 1B. Since the outer protective layer 8 serves to suppress the elastic force of the diametrically expandable portion 3 from exerting against the wire-stranded helical hollow tube 6, the outer protective layer 8 may be placed in the treatment tool 1B as the occcasion demands because the diametrically expandable portion 3 for the treatment tool 1B is initially liberated in the unrestricted free state.

The wire-stranded helical hollow tube 6 may be formed by twisting the wire coil elements 7 under the twist-resistant load, and thermally treated to remove the residual stress to insure a good rotation-following capability and straightness. Upon forming the wire-stranded helical hollow tube 6, a part or an entire portion of the wire coil elements may be made of an austenitic stainless steel, shape-memory alloy or superelastic alloy (e.g., Ni-Ti). A part of the outer protective layer 8 may be omitted from the wire-stranded helical hollow tube 6. Instead of the outer protective layer 8, a metallic tube (e.g., stainless steel tube and copper tube) may be used .

Upon forming the diametrically expandable portion 3, the the outer protective layer 8 is removed to expose the diametrically expandable portion 3, a part or an entire portion of the diametrically expandable portion exposed may be coated with the natural or urethane rubber. The treatment tool may be used with a medical catheter to retrievably capture the foreign matters back into the medical catheter when inserted into the blood vessel to slide the diametrically expandable portion against the vascular wall.

## Claims

1. A medical treatment tool (1, 1A, 1B) having a flexible linear wire forming a main linear wire portion (2) in the form of a hollow tube (6); an operational core elongation (4) slidably inserted into said main linear wire portion (2); a front end of said operational core elongation (4) being connected to a front end of said main linear wire portion (2), and a rear end of said operational core elongation (4) being connected to a hand access portion (5) placed at a rear end of said main linear wire portion (2); a diametrically expandable portion (3) provided in a proximity of a distal end portion of said main linear wire portion (2); and wherein:
said diametrically expandable portion (3) forms a basket-like configuration (B, B1, B2) which is shiftable into a diametrically shrunken configuration from said basket-like configuration (B, B1, B2) due to a relative sliding displacement between said operational core elongation (4) and said main linear wire portion (2;. **characterised in that:**
said wire-stranded helical hollow tube (6) of said main linear wire portion (2) is coated with an outer protective layer (8) extending from said hand-access portion (5) to said front end of said main linear wire portion (2); and **in that**
said diametrically expandable portion (3) is provided by separatively removing said outer protective layer (8) from said wire-stranded helical hollow tube (6) to leave it in a straight linear configuration; whereby in the diametrically shrunken configuration the diametrically expandable portion (3) has a diameter less than that of the wire-stranded helical hollow tube (6) coated with an outer protective layer (8).

2. The medical treatment tool (1, 1A, 1B) according to claim 1, wherein said wire-stranded helical hollow tube (6) of said diametrically expandable portion (3) is partly severed at its wire coil elements (7) to form a spoon-like configuration with an open end surface of said diametrically expandable portion (3) as a wire-lost region when said diametrically expandable portion (3) is expanded.

3. The medical treatment tool (1, 1A, 1B) according to claim 1 or 2, wherein a lock member (27, 27a) is provided to adjustably hold said diametrically expandable portion (3) alternatively as desired at a diametrically expanded position and at a diametrically shrunken position.

4. The medical treatment tool (1, 1A, 1B) according to claim 1, 2 or 3, wherein said hand access portion (5) forms a screw mechanism having a female (11, 17) thread provided at a side of said main linear wire portion (2) and a male thread portion (12, 18) provided at a side of said operational core elongation (4); and a dimensional length of wire coil elements (7) of said diametrically expandable portion (3) is approximately equal to an axial span appeared when said wire coil elements (7) are angularly twisted by 360 degrees.

5. The medical treatment tool (1, 1A, 1B) according to claim 1, 2, 3 or 4, wherein said hand access portion (5) forms said screw mechanism, and a stranding direction of said operational core elongation (4) is the same stranding direction of said wire-stranded helical hollow tube (6), and said stranding direction of said operational core elongation (4) is such oriented as to rotate in a direction when said operational core elongation (4) is manipulated to be pulled.

## Patentansprüche

1. Medizinisches Behandlungsinstrument (1, 1A, 1B) mit einem biegsamen geradlinigen Draht, welcher einen Hauptabschnitt (2) des geradlinigen Drahts in der Form eines hohlen Rohrs (6) bildet; einer betriebsfähigen Kernverlängerung (4), welche gleitbar in den Hauptabschnitt (2) des geradlinigen Drahts eingeführt ist; einem vorderen Ende der betriebsfähigen Kernverlängerung (4), welches mit einem vorderen Ende des Hauptabschnitts (2) des geradlinigen Drahts verbunden ist, und einem hinteren Ende der betriebsfähigen Kernverlängerung (4), welches mit einem Handzugriffabschnitt (5) verbunden ist, welcher an dem hinteren Ende des Hauptabschnitts (2) des geradlinigen Drahts angeordnet ist; einem diametral weitbaren Abschnitt (3), welcher in der Nähe eines distalen Endabschnitts des Hauptabschnitts (2) des geradlinigen Drahts vorgesehen ist; wobei:
der diametral weitbare Abschnitt (3) eine korbartige Konfiguration (B, B1, B2) bildet, die aufgrund einer relativen gleitenden Verschiebung zwischen der betriebsfähigen Kernverlängerung (4) und dem Hauptabschnitt (2) des geradlinigen Drahts von der korbartigen Konfiguration (B, B1, B2) in eine diametral geschrumpfte Konfiguration überführt werden kann, **dadurch gekennzeichnet, dass**
das mit einer Drahtlitze versehene spiralförmige hohle Rohr (6) des Hauptabschnitts (2) des geradlinigen Drahts, das mit einer äußeren Schutzschicht (8) beschichtet ist, sich von dem Handzugriffabschnitt (5) zu dem vorderen Ende des Hauptabschnitts (2) des geradlinigen Drahts erstreckt; und dass
der diametral weitbare Abschnitt (3) durch getrenntes Entfernen der äußeren Schutzschicht (8) von dem mit einer Drahtlitze versehenen spiralförmigen hohlen Rohr (6) vorgesehen ist, um es in einer geraden geradlinigen Konfiguration zu belassen, wodurch bei der diametral geschrumpften Konfiguration der diametral weitbare Abschnitt (3) einen geringeren Durchmesser als das mit der Drahtlitze versehene spiralförmige hohle Rohr (6) aufweist, welches mit einer äußeren Schutzschicht (8) beschichtet ist.

2. Medizinisches Behandlungsinstrument (1, 1A, 1 B) nach Anspruch 1, **dadurch gekennzeichnet, dass** das mit einer Drahtlitze versehene spiralförmige hohle Rohr (6) des diametral weitbaren Abschnitts (3) an seinen Drahtspulenelementen (7) teilweise durchtrennt ist, um eine löffelartige Konfiguration mit einer offenen Endfläche des diametral weitbaren Abschnitts (3) als Drahtverlustbereich zu bilden, wenn der diametral weitbare Abschnitt (3) geweitet ist.

3. Medizinisches Behandlungsinstrument (1, 1A, 1B) nach Anspruch 1 oder 2, **dadurch gekennzeichnet, dass** ein Verriegelungselement (27, 27a) vorgesehen ist, um den diametral weitbaren Abschnitt (3) alternativ nach Bedarf bei einer diametral weitbaren Position und einer diametral geschrumpften Position anpassbar zu halten.

4. Medizinisches Behandlungsinstrument (1, 1A, 1 B) nach Anspruch 1, 2 oder 3, **dadurch gekennzeichnet, dass** der Handzugriffabschnitt (5) einen Schraubenmechanismus mit einem Innengewinde (11, 17), welches an einer Seite des Hauptabschnitts (2) des geradlinigen Drahts vorgesehen ist, und einem Außengewindeabschnitt (12, 18), welcher an einer Seite der betriebsfähigen Kernverlängerung (4) vorgesehen ist, bildet; und eine dimensionale Länge der Drahtspulenelemente (7) des diametral weitbaren Abschnitts (3) in etwa gleich einer axialen Spanne ist, die auftritt, wenn die Drahtspulenelemente (7) im Winkel um 360° verdreht werden.

5. Medizinisches Behandlungsinstrument (1, 1A, 1 B) nach Anspruch 1, 2, 3 oder 4, **dadurch gekennzeichnet, dass** der Handzugriffabschnitt (5) den Schraubenmechanismus bildet und eine Litzenrichtung der betriebsfähigen Kernverlängerung (4) die gleiche Litzenrichtung wie die des mit einer Drahtlitze versehenen spiralförmigen hohlen Rohrs (6) ist und die Litzenrichtung der betriebsfähigen Kernverlängerung (4) so ausgerichtet ist, dass sie sich in eine Richtung dreht, wenn die betriebsfähige Kernverlängerung (4) so betätigt wird, dass sie gezogen wird.

## Revendications

1. Outil de traitement médical (1, 1A, 1B) comportant un fil linéaire souple formant une partie de fil linéaire principale (2) sous la forme d'un tube creux (6) ; une longueur de coeur opérationnel (4) insérée de façon à pouvoir coulisser à l'intérieur de ladite partie de fil linéaire principale (2) ; une extrémité avant de ladite longueur de coeur opérationnel (4) étant reliée à une extrémité avant de ladite partie de fil linéaire principale (2), et une extrémité arrière de ladite longueur de coeur opérationnel (4) étant reliée à une partie d'accès de main (5) disposée à une extrémité arrière de ladite partie de fil linéaire principale (2) ; une partie diamétralement extensible (3) disposée à proximité d'une partie d'extrémité distale de ladite partie de fil linéaire principale (2) ; et dans lequel :
ladite partie diamétralement extensible (3) forme une configuration analogue à un panier (B, B1, B2) qui peut être changée en une configuration diamétralement rétractée à partir de ladite configuration analogue à un panier (B, B1, B2) grâce à un déplacement de coulissement relatif entre de ladite longueur de coeur opérationnel (4) et ladite partie de fil linéaire principale (2) ; **caractérisé en ce que :**
ledit tube creux en serpentin à brins de fil (6) de ladite partie de fil linéaire principale (2) est revêtu d'une couche protectrice extérieure (8) s'étendant de ladite partie d'accès de main (5) à ladite extrémité avant de ladite partie de fil linéaire principale (2) ; et **en ce que** :
ladite partie diamétralement extensible (3) est réalisée en retirant séparément ladite couche protectrice extérieure (8) dudit tube creux en serpentin à brins de fil (6) de façon à laisser celui-ci sous une configuration linéaire droite ; grâce à quoi, dans la configuration diamétralement rétractée, la partie diamétralement extensible (3) a un diamètre inférieur à celui du tube creux en serpentin à brins de fil (6) revêtu d'une couche protectrice extérieure (8).

2. Outil de traitement médical (1, 1A, 1B) selon la revendication 1, dans lequel ledit tube creux en serpentin à brins de fil (6) de ladite partie diamétralement extensible (3) est partiellement coupé au niveau de ses élément de serpentin de fil (7) de façon à former une configuration en forme de cuiller avec une surface d'extrémité ouverte de ladite partie diamétralement extensible (3) constituant une région de fil perdu lorsque ladite partie diamétralement extensible (3) subit une expansion.

3. Outil de traitement médical (1, 1A, 1B) selon la revendication 1 ou 2, dans lequel un élément de blocage (27, 27a) est disposé de façon à maintenir de façon ajustable ladite partie diamétralement extensible (3) en alternance comme on le souhaite dans une position diamétralement étendue et dans une position diamétralement rétractée.

4. Outil de traitement médical (1, 1A, 1B) selon la revendication 1, 2 ou 3, dans lequel ladite partie d'accès de main (5) forme un mécanisme de vis comportant un filetage femelle (11, 17) disposé d'un côté de ladite partie de fil linéaire principale (2) et une partie de filetage mâle (12, 18) disposée d'un côté de ladite longueur de coeur opérationnel (4) ; et une longueur dimensionnelle d'éléments de serpentin de fil (7) de ladite partie diamétralement extensible (3) est approximativement égale à une étendue axiale apparaissant lorsque lesdits éléments de serpentin de fil (7) sont tordus de façon angulaire de 360 degrés.

5. Outil de traitement médical (1, 1A, 1B) selon la revendication 1, 2, 3 ou 4, dans lequel ladite partie d'accès de main (5) forme ledit mécanisme de vis, et une direction de brins de ladite longueur de coeur opérationnel (4) est la même direction de brins que celle dudit tube creux en serpentin à brins de fil (6), et ladite direction de brins de ladite longueur de coeur opérationnel (4) est orientée de façon à tourner dans une direction lorsque ladite longueur de coeur opérationnel (4) est manipulée pour être tirée.
